# EUROPEAN PATENT SPECIFICATION

(11) **EP 1 567 226 B1**
(45) Date of publication and mention of the grant of the patent: **20.08.2008**
(21) Application number: 03713112.5
(22) Date of filing: 17.03.2003
(51) Int. Cl.: A61N 2/00, A61N 2/02, A61N 1/06

(54) **APPARATUS FOR STIMULATING THE PHYSIOLOGICAL PROCESSES OF LIVING ORGANISM USING LIGHT WAVES ELECTROMAGNETIC INDUCTION AND THERMAL INTERACTION**
GERÄT ZUR STIMULATION DER PHYSIOLOGISCHEN PROZESSE DES LEBENDEN ORGANISMUS MIT LICHTWELLEN, ELEKTROMAGNETISCHER INDUKTION UND THERMISCHER INTERAKTION
APPAREIL DE STIMULATION DE PROCESSUS PHYSIOLOGIQUES D'ORGANISMES VIVANTS AU MOYEN D'ONDES LUMINEUSES, D'UNE INDUCTION ELECTROMAGNETIQUE ET D'UNE INTERACTION THERMIQUE

(30) Priority: 15.11.2002 PL 35714902
(43) Date of publication of application: 31.08.2005
(73) Proprietor: Piotrowicz, Mariusz, 32-500 Chrzanow (PL)
(72) Inventor: Piotrowicz, Mariusz, 32-500 Chrzanow (PL)
(74) Representative: Drelichowski, Henryk
(86) International application number: PCT/PL2003/000026
(87) International publication number: WO 2004/045715

(56) References cited:
- US-A- 4 471 787
- US-A- 5 000 178

## Description

The subject of the invention is an apparatus for stimulating the physiological processes of living organisms using light waves, electromagnetic waves and thermal interaction, simultaneously bringing both the cells and molecules of living organism into an energetically richer excited state.

Various types of apparatuses emitting variable electromagnetic fields, apparatuses emitting thermal waves, as well as apparatuses emitting light waves are well known.

United States of America patent specification no. 4,685,462 concerns an apparatus for the treatment of hypothermia using a magnetic field. The apparatus in question is equipped with two spiral induction coils coupled with an RF generator through an automatic frequency tuning system which includes condensers and induction coils. The spiral coils are located in a non-conducting cylinder that constitutes an element of the shield. One of the coils receives a signal from the RF generator with a frequency tuned to the resonance, while the other - by mutual induction.

United States of America patent specification no. 5,344,384 concerns an apparatus for magnetotherapy that is stimulated by a magnetic field generated periodically from coils with a field frequency ranging from 10 to 90 Hz when required. This apparatus functions as a stimulator for sick parts of the human body. It comprises a number of flexible elements that are permanently connected with each other. Each of these elements has an internal magnetic field generator, made up of a high-power coil that fills nearly the entire flexible element. These elements may be permanently connected to create apparatuses in the form of mats, belts and compresses placed on sick parts of the human body.

United States of America patent specification no. 5,453,074 concerns an apparatus for electromagnetic therapy that is intended for human beings and improves the general physical condition thereof. It is made up of a flat base terminating in a headrest and covered with a movable part, which is shorter than the base and moves along it. This apparatus may generate electromagnetic field - safe for the patient - for individual parts of the human body and the head over short periods of time; the fields are generated by electromagnetic generators located in the apparatus' cylinders.

German utility model description no. 299 19 950 concerns a therapeutic lamp for the treatment of individual parts of the human body by means of a concentrated electromagnetic field. This lamp has a concentrated system of spirals located in a narrow housing, emitting an electromagnetic field ensuring extremely limited interaction with specific, small part of the human body.

Various types of apparatuses emitting light waves of varying lengths and with relatively high emission powers, ranging from 30 to 300 W, are well known.

Polish patent specification no. 177426 concerns a therapeutic lamp for biostimulation utilising polarised light, which includes a spot-light source with a power rating ranging from 30 to 300 W that is located in the focus of a reflector in the shape of a paraboloid of revolution; it contains a light filter plate and integral plastic housing made up of two tubular parts with identical circular sections, which are connected with each other at an angle of 114°.

Polish patent specification no. 181716 concerns an apparatus for photodynamic irradiation with a housing fitted with a lamp, a reflector surrounding the lamp, a set of filters installed on the path of the light beam, a light outlet located behind the set of filters, a dosing device for the precise measuring of radiation energy provided to the patient by the apparatus, and an electronic range-finder for measuring the irradiation distance and for utilising such measurements when setting the required irradiation distance.

United States of America patent specification no. 6450941 concerns a hair-care and drying apparatus that stimulates body cells with pulsating electromagnetic radiation; it is powered by current pulses with a frequency ranging from 200 Hz to 20000 Hz and a current pulse amplitude between 15 and 25 V. The pulse duration ranges from 2 to 200 microseconds. At the same time, infrared radiation of three wavelengths - 600 nm, 900 nm and 1200 nm - and blue light with a wavelength of 400 nm, generated by semiconductor diodes or laser diodes, acts on the hair. The commencement of pulsating electromagnetic and visible radiation is accompanied by the activation of a blower supplying hot air with a temperature of 37° C, which dries the hair. The apparatus may act on the hair and skin of the head for only a relatively short time, from a few to a few dozen minutes. This time is limited by the complete drying of the hair by the hot air supplied by the electric blower.

The disadvantage of all of the abovementioned apparatuses and methods applied for living organisms consists in the fact that there exists no apparatus enabling the optional connection of electromagnetic and magnetic field emission with the emission of light waves of varying lengths and/or with the emission of heat onto the living organism. For safety reasons, the apparatuses hitherto used may be used only for relatively short periods of time, ranging from a few to a few dozen minutes. This brevity of application is the result of excessive values of magnetic induction emission or the high values of the electromagnetic field acting on the basis of pulses and the high power of lamps emitting light waves, which causes the overheating of the parts of the human body exposed to radiation. When used for longer periods, the apparatuses hitherto known - utilising current frequencies from 200 to 20000 Hz - exert a negative influence on many of the reactions occurring in living organisms.

It is commonly known that the living organism adapts favourably in processes with very low electromagnetic field values and negligible powers of light wave emitters acting thereon, together with an optimal temperature acting on the whole organism at the same time. Such processes should influence the living organism on a long-term basis, while in certain instances - even continuously over extended periods of time. In the case of living organisms, sleep or rest are the times most appropriate for such types of interaction.

The objective of the invention consisted in constructing an apparatus that would ensure the safety of stimulation and the course of living processes, and at the same time would make it possible for a living organism to be influenced by an electromagnetic field with a low value of electromagnetic induction emission, this being accompanied by the negligible power of lamps used for the emission of light waves and the low, non-forced thermal emission over an unlimited period of time on the organism. An apparatus of this type would also favourably influence the organism in the event of advanced ailments, where the immunological system is already heavily burdened and is unable to withstand the momentary, strong action of existing apparatuses.

In accordance with the invention, the apparatus for stimulating the physiological processes of living organisms has a spatial form, limited by two parallel or nearly parallel planes. Between these planes there is a space with an appropriately selected height, which may be limited by side walls. The apparatus is made up of a number of supports of equal height and optional shape. These supports are connected with the upper plane of the apparatus, this being thinly woven material or - preferably - a mesh of glass fibre or any other material, inhibiting the development of bacteria. In accordance with the invention, the upper side of the thinly woven material is lined with an insulating thermal material, advantageously elastic and with fungicidal and bactericidal properties. In the lower parts of the supports, immediately above their plane of foundation, there are installed emitters of electromagnetic waves, advantageously coils and/or groups of coils emitting an electromagnetic field with a frequency ranging from 10 Hz to 100 Hz and with electromagnetic induction ranging from 0.001 µT to 80 µT. Preferably, the frequency of the electromagnetic field should range from 50 to 60 Hz, while the electromagnetic induction - from 0.01 µT to 5.00 µT. The electromagnetic wave emitters also emit the required quantity of heat. The electromagnetic wave emitters are selected in such a way, so that at the level of the upper plane of the apparatus, slightly above the thinly woven material or at the level thereof, at each and every point of the upper plane of the apparatus - in accordance with the invention - it is possible to obtain any value of electromagnetic induction within the range 0,001 µT - 80 µT. In order to enable the penetration of air and thermal emission, the insulating thermal material has freely placed and optionally sized openings which let through air. These openings may have the shape of elongated slits. They may also be round or ellipsoidal, with varying or identical diameters, or create slits around honeycombed regular polygons made of the insulating thermal material. The slits or openings in the insulating thermal material contain light wave emitters, which emit waves with lengths ranging from 380 nm to 630 nm and a generated frequency from 0.5 MHz to 100 MHz. It is advantageous for the emitted light waves to have a generated frequency within the range of 0.8 MHz to 1.2 MHz. The light wave emitters in the openings of the insulating thermal material are installed in quads or in series, and comprise emitters emitting light with an identical length of the light wave or with light wave lengths varying within the range 380 nm - 630 nm. In these slits or openings in the insulating thermal material there are installed from one to many emitters of light waves, depending on requirements; it is favourable for the number of emitters to total between 1 and 8. It is important for the light wave emitters to occupy 2/3 of the length of the apparatus, or the central part thereof. The light wave emitters are low-power units up to 100 mW, and it is advantageous if they are light diodes with a power from 20 mW to 50 mW. The electromagnetic wave emitters are powered by an alternating current with a voltage that is safe for living organisms, i.e. ranging from 6 to 24 V. The strength of the electromagnetic field emitted by the electromagnetic wave emitters is regulated by changing the intensity of the current flowing through selected electromagnetic wave emitters. In accordance with the invention, the apparatus is fitted with sensors and devices for the control, measurement and regulation of temperature in the vicinity of the upper plane. It may also be equipped with sensors and devices for the continuous measurement and regulation of the value of emitted electromagnetic induction, at each and every point of the apparatus' upper plane. Both the sensors and the apparatuses for the measurement and regulation of emitted values of electromagnetic induction and the value of heat emitted are connected to the control system of the apparatus. The control system of the apparatus is fitted with a generator of the frequencies of emitted light waves, this enabling the trouble-free adapting of their frequencies to current operating requirements. Because the control system of the apparatus is fitted with a processor, it is possible to continuously monitor the values of electromagnetic induction emissions, the values of heat emission and light emission by means of a personal computer. In order to enhance operator comfort, the invented apparatus may be divided along its longer side into two or more elements permanently connected with each other, enabling the assembly or disassembly thereof.

In accordance with the invention, a variant of the apparatus may comprise elements of varying shapes and sizes, usually in the form of belts, compresses or other, fixed to individual parts of the living creature. These consist of thermal insulating material with freely shaped openings installed on appropriately shaped thinly woven material. Light wave emitters are installed in these openings. The surface lined with the thermal insulating material adheres directly to the part of the body upon which the appropriately shaped element is placed. The opposite side of the appropriately shaped thinly woven material may have an empty space with an appropriately selected thickness, limited from the outside by a material enabling the controlled, natural penetration of air; to the inside thereof there are fixed electromagnetic wave emitters that emit an electromagnetic field and the required quantity of heat. The entire apparatus - both with installed electromagnetic wave emitters and installed light wave emitters - is powered by batteries or accumulators. The batteries or accumulators also power the system for measuring, controlling and regulating the emission of media by the apparatus. This variant makes it possible to simultaneously act by means of electromagnetic emission, light emission and heat emission on selected parts of the body of a living organism, without hindering the freedom of movement thereof.

In accordance with the invention, another advantageous variant of the apparatus is one with dimensions selected for various objectives used to sit or lie on, including car seats.

In accordance with the invention, a different variant of the invention is a mattress. The lower plane of the mattress is permanently connected with the side walls. The bottom part of the mattress and its side walls are lined with a leakproof non-permeable material. The internal part of the mattress bottom or the area just above the bottom carries emitters of electromagnetic waves with an electromagnetic induction in the range 0.001 µT - 80 µT, which also emit the required quantity of heat. It is advantageous if the electromagnetic wave emitters are placed beneath/or between two fabrics or materials that allow for the passage of heat radiation. The upper layer of the apparatus is a thinly woven material. In accordance with the invention, the thinly woven material comprising the upper layer of the apparatus is lined with an insulating thermal material. In order to enable the penetration of air and thermal emission, the insulating thermal material has freely spaced and optionally shaped air-permeable openings, in which there may be installed light wave emitters. The side walls of the apparatus may have ventilating holes, opened and closed manually or automatically whenever it becomes necessary to lower or increase the thermal value emitted by the electromagnetic wave emitters in the direction of the upper plane of the apparatus. The clear area inside the apparatus, in order to enhance the comfort of the user, may be fitted - this between the bottom and thinly woven material - with a spring-based upholstery structure, with a clear area left around the upholstery springs in order to enable ventilation and the circulation of media within the apparatus and the emission thereof towards the upper plane. The external plane of the bottom and the sides of the apparatus have - typical for mattresses - an easy to remove upholstery, which is partially turned up above the upper edge of the sides on to the insulating material placed above, this in such a way as not to cover the slits or openings in the insulating material.

One of the advantages of the apparatus as presented in accordance with the invention consists in the fact that it is possible to simultaneously act on the living organism by the emission of an electromagnetic field with low values, by the emission of low-power light waves, and also natural heat emission. The low values of media emitted mean that living organisms can freely remain in their reaction field without any time limits. The living organism utilising the apparatus as presented in accordance with the invention is exposed neither to any hazardous high frequencies of the currents flowing through the apparatus, nor to the thermal overheating of skin or internal cells. The apparatus makes it possible to maintain the stable temperature of the living organism, and prevents its overheating or sweating. An additional advantage of the apparatus is that it ensures optimal sleeping conditions, and thus even over a short period of sleep it is possible to ensure the improved regeneration of living organisms. When the apparatus is adapted to seats, and in particular to car seats, drivers benefit from higher levels of concentration.

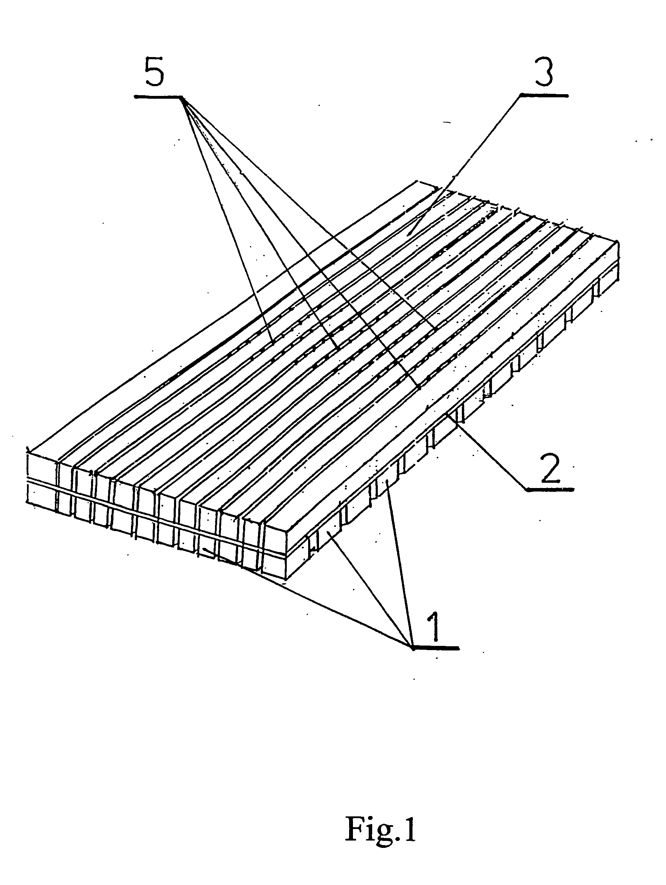

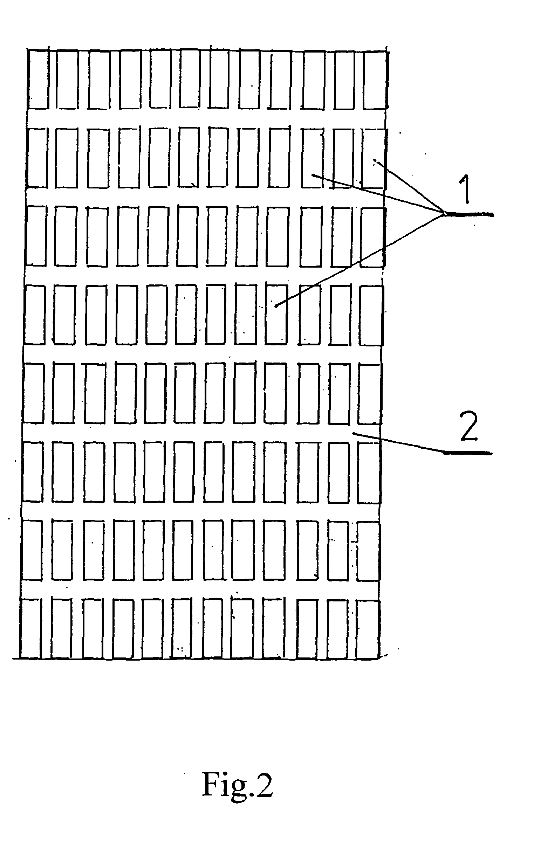

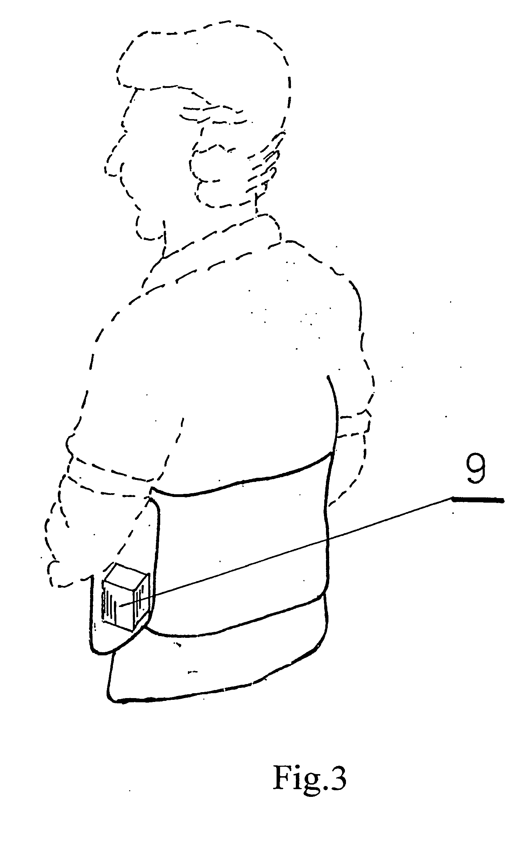

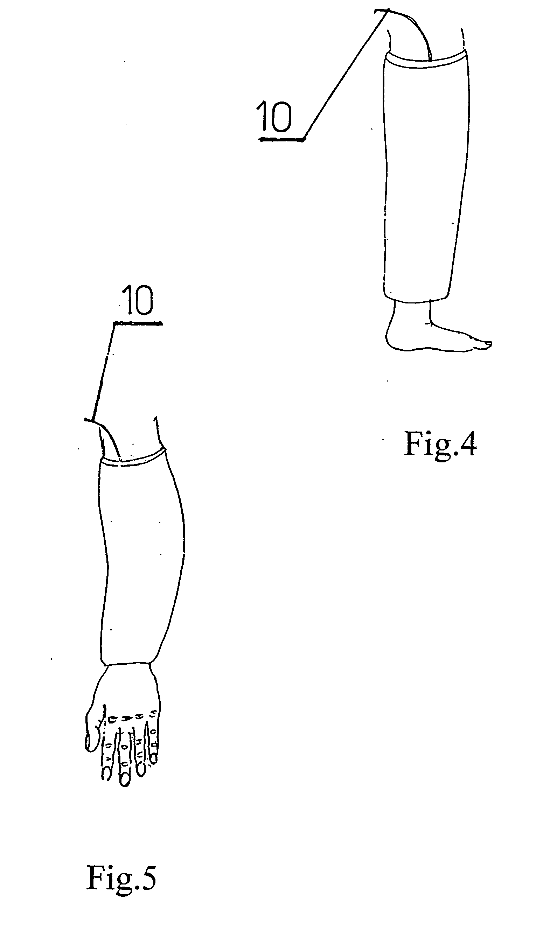

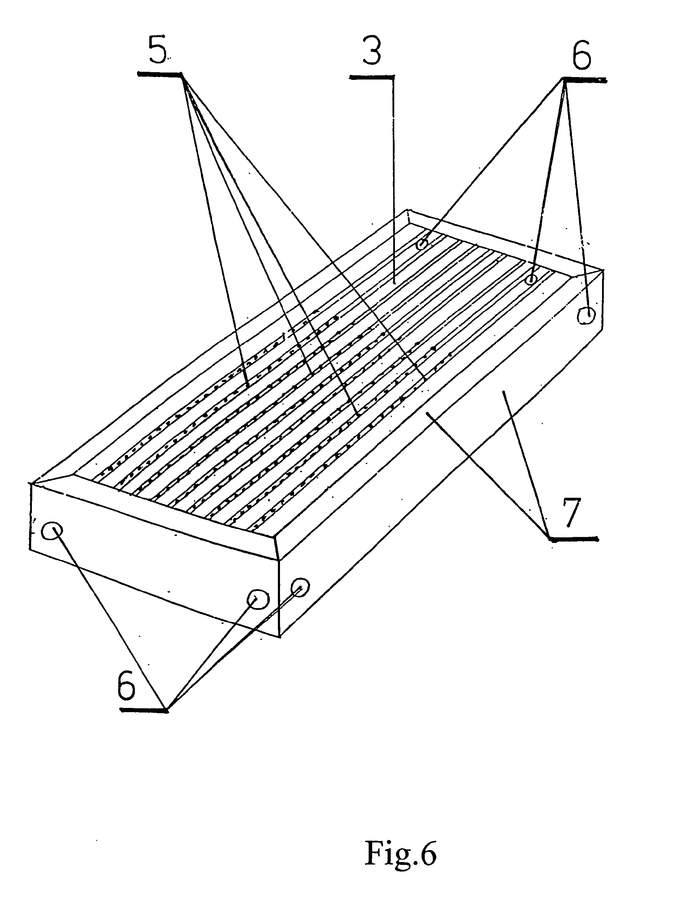

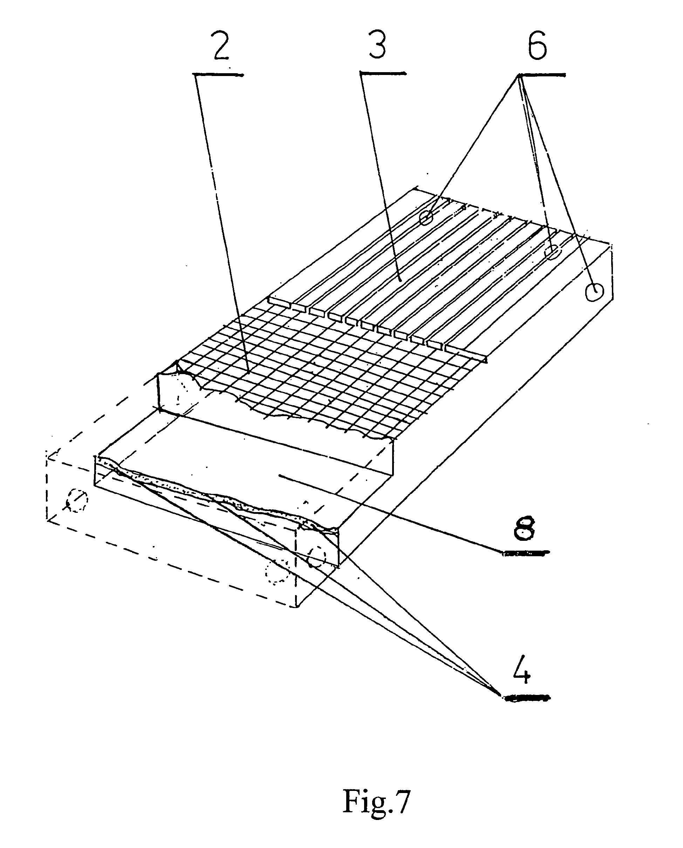

In accordance with the invention, the apparatus for stimulating the physiological processes of living organisms has been presented on the basis of the following manufactured example, where Fig.1 presents the apparatus in accordance with the invention with elongated openings, Fig.2 presents the exemplary spacing of the supports of the apparatus, Fig.3 presents an exemplary form of the apparatus, in the shape of a strip, Fig.4 presents an exemplary form of the apparatus in the shape of a leg compress, Fig.5 presents an exemplary form of the apparatus in the shape of a armband, Fig.6 presents the apparatus in the shape of a mattress, Fig.7 presents the apparatus in the shape of a mattress (axonometric section).

In accordance with the invention, the apparatus for stimulating the physiological processes of living organisms, in the shape of a flat rectangular prism, consists of supports 1. to which there are attached electromagnetic wave emitters. The supports 1 are permanently connected to the bottom part of the thinly woven material 2. The upper plane of the thinly woven material 2 is lined with an insulating material that has elongated openings, in which there are installed light wave emitters 5. The variant of the apparatus in the form of a band has a feeder cable and control device 9. The variants of the apparatus in the form of an armband and headband are connected by cable 10 with the feeder cable and control device 9. The variant of the apparatus in the shape of a mattress has light wave emitters 5 fitted in the openings of the insulating material 3. Openings 6 are situated in the side walls of the mattress-shaped apparatus and at one of the shorter edges, in the thermal insulating material. The electromagnetic wave emitters 4 are installed between the two fabrics 8.

## Claims

1. The apparatus for stimulating the physiological processes of living organisms in the form of a rectangular prism, containing between the bottom and the upper plane a clear area enabling the emission of electromagnetic and thermal waves, with coils fitted at the bottom, between two fabrics enabling the permeation of heat radiation, **characterized by** its spatial form, limited by two parallel or nearly parallel planes, comprising a number of supports (1) with an identical height and optional shape, permanently connected with the upper plane of the apparatus, this comprising a thinly woven material (2), lined from above with an insulating thermal material (3), whereas the insulating thermal material (3) has freely spaced and optionally shaped openings in which there are installed light wave emitters (5) that emit light waves with a length ranging from 380 nm to 630 nm, frequency from 0.5 MHz to 100 MHz and power of up to 100 mW, while the supports (1) in their lower part have installed electromagnetic wave emitters (4), selected in such a way so that at the level of the upper plane of the apparatus, slightly above the thinly woven material or at the level thereof, at each and every point of the upper plane of the apparatus it is possible to obtain any value of electromagnetic induction within the range 0.001 µT - 80 µT, with a frequency of 20 Hz to 80 Hz, simultaneously emitting a specific quantity of heat, whereas the equipment is powered by an alternating current with a voltage that is safe for living organisms, ranging from 6 to 24 V, and is connected to the control system of the apparatus, containing a generator of the frequencies of emitted light waves.

2. The apparatus, pursuant to claim 1, **characterized by** the fact that the electromagnetic wave emitters (4) emit waves with an electromagnetic induction within the range of 0.01 µT to 5.00 µT and with a frequency ranging from 40 Hz to 60 Hz.

3. The apparatus, pursuant to claim 1, **characterized by** the fact that the thinly woven material (2) is a mesh made from fibre glass or any other material inhibiting the development of bacteria.

4. The apparatus, pursuant to claim 1, **characterized by** the fact that the insulating thermal material (3) is elastic and has fungicidal and bactericidal properties.

5. The apparatus, pursuant to claim 1, **characterized by** the fact that the freely spaced openings in the insulating thermal material (3) have the shape of elongated slits or circles and/or ellipsoids with differing or identical diameters, or form slits around honeycombed regular polygons.

6. The apparatus, pursuant to claim 5, **characterized by** the fact that the light wave emitters (5) emit light waves with a frequency ranging from 0.8 MHz to 1.2 MHz and are located in series or in quads, in freely selected quantities.

7. The apparatus, pursuant to claim 6, **characterized by** the fact that the number of light wave emitters (5) totals between 1 and 8.

8. The apparatus, pursuant to claim 7, **characterized by** the fact that the light wave emitters are situated at 2/3 of the length of the apparatus or in the central part of the upper open plane of the apparatus.

9. The apparatus, pursuant to claim 6, **characterized by** the fact that the light wave emitters (5) are diodes with a power of 20 mW to 50 mW.

10. The apparatus, pursuant to claim 1, **characterized by** the fact that it has the shape of belts, compresses or other, attached to individual parts of the human body or as the lining of other objects used to sit or lie on.

11. The apparatus, pursuant to claim 1, **characterized by** the fact that it constitutes a mattress, the bottom of which is permanently connected with the side walls, whereas the bottom and interior parts of the side walls are lined with a leakproof non-permeable material, while the internal part of the mattress bottom or the area just above the bottom carries emitters of electromagnetic waves (4) and heat emitters, while the upper plane of the apparatus is a thinly woven material (2), lined from above with an insulating thermal material (3) that has freely spaced and optionally shaped openings, in which there are located light wave emitters (5), whereas the side walls of the apparatus have ventilation holes (6), opened and closed manually or automatically, and the clear area inside the apparatus is fitted - this between the bottom and thinly woven material - with a spring-based upholstery structure, with a clear area left around the upholstery springs.

12. The apparatus, pursuant to claim 11, **characterized by** the fact that the electromagnetic wave emitters (4) are coils and/or groups of coils.

13. The apparatus, pursuant to claim 12, **characterized by** the fact that the coils and/or groups of coils (4) are placed beneath/or between two fabrics or materials (8) enabling the permeation of heat radiation.

## Patentansprüche

1. Gerät in Quaderform zur Stimulation der physiologischen Prozesse von lebenden Organismen, das zwischen dem Boden und der oberen Fläche über freien Raum für die Ermöglichung der Emission der elektromagnetischen und thermischen Wellen verfügt, an dessen Boden zwischen zwei für Wärmestrahlung durchlässigen Geweben Spulen installiert sind, und das **dadurch gekennzeichnet ist, dass** es eine durch zwei Parallelen oder den Parallelen angenäherte Ebenen beschränkte räumliche Form hat und aus einer Reihe von Auflagern (1) mit einheitlicher Höhe und mit beliebiger Form fest mit der oberen Fläche des Gerätes verbunden sind, die ein thermischer Isolierstoff mit dünner Bindung (2) ist, der von oben mit thermischem Isolierstoff (3) ausgekleidet ist, wobei der thermische Isolierstoff (3) beliebig angeordnete Öffnungen in beliebiger Form aufweist, in denen Lichtwellenstrahler (5) montiert sind, die Lichtwellen mit der Länge von 380 nm bis 630 nm, mit generierter Frequenz von 0,5 MHz bis 100 MHz und der Leistung von 100 mW ausstrahlt, wobei die in dem unteren Teil der Auflager eingebauten Strahler elektromagnetischer Wellen (4) so ausgewählt sind, dass es in Höhe der oberen Fläche des Gerätes, etwas über dem montierten Stoff mit dünner Bindung oder auf seinem Niveau, an jedem beliebigen Punkt der oberen Fläche des Gerätes ein beliebiger Wert der elektromagnetischen Induktion im Bereich vom 0,001 µT bis 80 µT und mit Frequenz von 10 Hz bis 100 Hz erzielt werden kann, die gleichzeitig eine bestimmte Wärmemenge emittieren, wobei das Gerät mit dem Wechselstrom mit der Grenzspannung im Bereich von 6V bis 24V eingespeist wird, und an das Steuerungssystem des Gerätes angeschlossen ist, das mit dem Frequenzenerator für die emittierten Lichtwellen ausgerüstet ist.

2. Das Gerät ist, gemäß Anspruch 1, **dadurch gekennzeichnet, dass** die Strahler der elektromagnetischen Wellen (4) Wellen mit der elektromagnetischen Induktion im Bereich von 0,01 µT do 5,00 µT und mit der Frequenz von 50 Hz bis 60 Hz emittieren.

3. Das Gerät ist, gemäß Anspruch 1, **dadurch gekennzeichnet, dass** der Stoff mit dünner Bindung (2) ein Netz aus Glasfasern oder einem anderen geeigneten Material ist, das die Entwicklung von Bakterien hemmt.

4. Das Gerät ist, gemäß Anspruch 1, **dadurch gekennzeichnet, dass** der thermische Isolierstoff (3) elastisch ist und bakterizide und fungizide Eigenschaften aufweist.

5. Das Gerät ist, gemäß Anspruch 1, **dadurch gekennzeichnet, dass** die beliebig in den thermischen Isolierstoff (3) angebrachten Öffnungen die Form von länglichen Schlitzen oder Kreisen und/oder Ellipsen mit unterschiedlichem oder einheitlichem Durchmesser haben, oder Schlitzen um die regelmäßigen Vielecke bilden, die in Form der "Bienenwabe" verlegt sind.

6. Das Gerät ist, gemäß Anspruch 5, **dadurch gekennzeichnet, dass** die in den Schlitzen oder Öffnungen des thermischen Isolierstoffes (3) angebrachten Lichtwellenstrahler (5) die Lichtwellen mit generierter Frequenz von 0,8 MHz bis 1,2 MHz emittieren und in Reihen oder in Bündeln in beliebiger Anzahl angeordnet sind.

7. Das Gerät ist, gemäß Anspruch 6, **dadurch gekennzeichnet, dass** die Anzahl der Lichtwellenstrahler (5) sich insgesamt zwischen 1 bis 8 bewegt.

8. Das Gerät ist, gemäß Anspruch 7, **dadurch gekennzeichnet, dass** die Lichtwellenstrahler (5) auf einem Punkt in Höhe von 2/3 der Länge des Gerätes oder im mittleren Teil seiner oberen, offenen Fläche angebracht sind.

9. Das Gerät ist, gemäß Anspruch 6, **dadurch gekennzeichnet, dass** die Lichtwellenstrahler (5) Lichtdioden mit der Leistung von 20 mW bis 50 mW sind.

10. Das Gerät ist, gemäß Anspruch 1, **dadurch gekennzeichnet, dass** es die Form von Gürteln, Kompressen oder derartigem hat, die an bestimmten Körperteilen des lebenden Organismus befestigt werden oder für die Auskleidung anderer Gegenstände zum Sitzen oder Liegen dienen.

11. Das Gerät ist, gemäß Anspruch 1, **dadurch gekennzeichnet, dass** es eine Matratze ist, derer Boden mit den seitlichen Wänden fest verbunden ist, wobei der Boden und die seitlichen Wände an der Innenseite mit einem dichten, luftundurchlässigen Stoff ausgekleidet sind, an der Innenseite des Matratzebodens hingegen oder gleich über dem Matratzeboden die Strahler der elektromagnetischen Wellen (4) und der Wärmeemission montiert sind, die obere Fläche des Gerätes ist hingegen ein Stoff mit dünner Bindung (2), der von oben mit thermischem Isolierstoff (3) ausgekleidet ist, der beliebig angeordnete Öffnungen beliebiger Form hat, in denen die Lichtwellenstrahler (5) angebracht sind, wobei die seitlichen Wände über Lüftungsöffnungen (6) verfügen, die von hand oder automatisch geschlossen und geöffnet werden und der freie Raum innerhalb des Gerätes zwischen dem Boden und dem Stoff mit dünner Bindung über eine Feder unetrstützte Polsterkonstruktion verfügt, bei der um die Polsterfedern ein Freiraum gelassen wurde.

12. Das Gerät ist, gemäß Anspruch 11, **dadurch gekennzeichnet, dass** die Strahler der elektromagnetischen Wellen (4) Spulen und/oder Spulensätze sind.

13. Das Gerät ist, gemäß Anspruch 12, **dadurch gekennzeichnet, dass** die Strahler der elektromagnetischen Wellen (4) unter/oder zwischen zwei Geweben oder Stoffen (8) angebracht sind, die thermische Strahlung durchlassen.

## Revendications

1. L'appareil de stimulation de processus physiologiques d'organismes vivants ayant la forme d'un prisme rectangulaire, présentant entre ses plans inférieur et supérieur une surface dégagée permettant l'émission d'ondes électromagnétiques et thermiques, doté de bobines installées au niveau de la surface inférieure, entre deux membranes permettant la perméation de la radiation calorifique, **caractérisé par** sa forme spatiale, présentant à ses extrémités deux plans parallèles ou presque parallèles, comprenant un ensemble d'appuis (1)de même hauteur et de différentes formes au choix, connectés en permanence au plan supérieur de l'appareil, celui-ci comprenant une membrane finement tissée (2), doublée depuis la partie supérieure d'une membrane thermique isolante (3), ladite membrane thermique isolante (3) présentant des ouvertures espacées de manière aléatoire et de forme déterminée au choix, dans lesquelles sont installés des émetteurs d'ondes lumineuses (5) émettant des ondes lumineuses d'une longueur de 380 nm à 630 nm, dont la fréquence est de 0,5 MHz à 100 MHz et d'une puissance pouvant atteindre 100 mW; les appuis (1) étant quant à eux dotés, au niveau de leur partie inférieure, d'émetteurs d'ondes électromagnétiques (4), sélectionnés de manière à ce qu'au niveau du plan supérieur de l'appareil, légèrement au-dessus de la membrane finement tissée ou au niveau de celle-ci, en tout point du plan supérieur de l'appareil, il soit possible d'obtenir toute valeur d'induction électromagnétique oscillant entre 0,001 µT et 80 µT, d'une fréquence de 20 Hz à 80 Hz, émettant simultanément une quantité déterminée de chaleur; l'équipement étant alimenté en courant alternatif d'une tension sans danger pour les organismes vivants (de 6 à 24 V) et connecté au système de commande de l'appareil, doté d'un générateur de fréquences des ondes lumineuses émises.

2. L'appareil, aux termes de la revendication 1, **caractérisé par le fait que** les émetteurs d'ondes électromagnétiques (4) émettent des ondes dont l'induction électromagnétique se situe entre 0,01 µT et 5,00 µT et d'une fréquence de 40 Hz à 60 Hz.

3. L'appareil, aux termes de la revendication 1, **caractérisé par le fait que** la membrane finement tissée (2) est un grillage fabriqué à partir de fibre de verre ou de tout autre matériau empéchant la croissance de bactéries.

4. L'appareil, aux termes de la revendication 1, **caractérisé par le fait que** la membrane thermique isolante (3) est élastique et présente des propriétés fongicides et bactéricides.

5. L'appareil, aux termes de la revendication 1, **caractérisé par le fait que** les ouvertues espacées de manière aléatoire au niveau de la membrane thermique isolante (3) ont la forme de fentes allongées ou de cercles et/ou d'ellipsoïdes de diamètres différents ou identiques, ou forment des fentes autour de polygones réguliers percés.

6. L'appareil, aux termes de la revendication 5, **caractérisé par le fait que** les émetteurs d'ondes lumineuses (5) émettent des ondes lumineuses d'une fréquence de 0,8 MHz à 1,2 MHz et disposés en série ou par quatre, en quantités déterminées au choix.

7. L'appareil, aux termes de la revendication 6, **caractérisé par le fait que** le nombre d'émetteurs d'ondes lumineuses (5) se situe entre 1 et 8.

8. L'appareil, aux termes de la revendication 7, **caractérisé par le fait que** les émetteurs d'ondes lumineuses sont situés au niveau des 2/3 de la longueur de l'appareil ou au niveau de la partie centrale du plan ouvert supérieur de l'appareil.

9. L'appareil, aux termes de la revendication 6, **caractérisé par le fait que** les émetteurs d'ondes lumineuses (5) sont des diodes d'une puissance de 20 mW à 50 mW.

10. L'appareil, aux termes de la revendication 1, **caractérisé par le fait qu'**il présente la forme de ceintures, compresses ou autres, fixées sur des parties du corps humain considérées isolément, ou constitue le revêtement d'autres objets sur lesquels il est possible de s'asseoir ou de s'étendre.

11. L'appareil, aux termes de la revendication 1, **caractérisé par le fait qu'**il constitue un matelas dont le fond est relié en permanence aux parois latérales dont les parties inférieure et interne sont doublées d'une membrane étanche et imperméable; la partie intérieure du fond du matelas ou la surface juste au-dessus du fond porte des émetteurs d'ondes électromagnétiques (4) ainsi que des émetteurs de chaleur, tandis que le plan supérieur de l'appareil est formé d'une membrane finement tissée (2), doublée depuis la partie supérieure d'une membrane thermique isolante (3), ladite membrane thermique isolante (3) présentant des ouvertures espacées de manière aléatoire et de forme déterminée au choix, dans lesquelles sont installés des émetteurs d'ondes lumineuses (5); les parois latérales de l'appareil présentent des orifices de ventilation (6) pouvant être ouverts ou fermés manuellement ou automatiquement, et la surface dégagée à l'intérieur de l'appareil comporte - entre le fond et la membrane finement tissée - une structure de rembourrage à ressorts, des zones dégagées étant prévues entre les ressorts de rembourrage.

12. L'appareil, aux termes de la revendication 11, **caractérisé par le fait que** les émetteurs d'ondes électromagnétiques (4) sont des bobines ou groupes de bobines.

13. L'appareil, aux termes de la revendication 12, **caractérisé par le fait que** les bobines et/ou groupes de bobines (4) sont placés sous/ou entre deux structures ou membranes (8) permettant la perméation de la radiation calorifique.
